# EUROPEAN PATENT APPLICATION

(11) **EP 1 856 973 A1**
(43) Date of publication of application: **21.11.2007**
(21) Application number: 06715289.2
(22) Date of filing: 06.03.2006
(51) Int. Cl.: A01N 1/02

(54) **ORGAN FUNCTION MAINTAINING AND AMELIORATING SOLUTION**

(30) Priority: 04.03.2005 JP 2005060976; 04.03.2005 JP 2005060992
(71) Applicant: National Universiy Corporation Tokyo Medical and Dental University, Tokyo 113-8510 (JP); Minophagen Pharmaceutical Co., Ltd., Tokyo 107-0052 (JP)
(72) Inventor: KUDO, Atsushi National University Corporation, Bunkyo-ku, Tokyo 1138510 (JP); ARII, Shigeki National University Corporation, Bunkyo-ku, Tokyo 1138510 (JP); WAKE, Kenjiro, Tokyo 1800002 (JP); SAKANO, Isao, Tokyo 1940042 (JP)
(74) Representative: HOFFMANN EITLE
(86) International application number: PCT/JP2006/304269
(87) International publication number: WO 2006/093314

(57) **Abstract**

It is intended to provide an organ function maintaining or ameliorating solution, which is effective for suppressing the depression of an organ provided for transplantation and an organ preservation method using the organ function maintaining or ameliorating solution.

The present invention relates to an organ function maintaining or ameliorating solution for maintaining or ameliorating the functions of an organ provided for organ transplantation, comprising a pharmacologically active substance comprising glycyrrhizic acid, glycyrrhetinic acid or a pharmacologically acceptable salt or derivative thereof in an organ preservation solution. The present invention also relates to a method of preserving an organ for organ transplantation comprising perfusing the organ function maintaining or ameliorating solution to the organ isolated from the donor to cause ischemia and immersing the organ in the organ function maintaining or ameliorating solution during the time until the organ is transplanted into a recipient.

## Description

### TECHNICAL FIELD

The present invention relates to an organ function maintaining or ameliorating solution for maintaining or ameliorating the function of an organ, which is isolated for organ transplantation. More specifically, it relates to an organ function maintaining or ameliorating solution comprising a pharmacologically active substance comprising glycyrrhizic acid, glycyrrhetinic acid or a pharmacologically acceptable salt or derivative thereof in an organ preservation solution.

### BACKGROUND ART

With the recent increase in organ transplantation cases, a technique for preserving an isolated organ in the state of maintaining or ameliorating the functions of the isolated organ becomes more and more important. For example, although it is favorable to transplant a liver for transplantation into an organ receiver (a recipient) immediately after the surgical isolation, a brain-dead organ provider (a donor) usually appears in a place distant from the recipient. Therefore, the isolated liver should be rapidly transported by air transportation and so on. It is well known that an isolated organ is required to be transported and preserved over several hours to 24 hours in some cases. In the case of living donor liver transplantation, it may be also required that the isolated liver should be treated by cold preservation ischemic perfusion followed by cold preservation to keep the isolated liver fresh until the transplantation into a recipient.

An isolated liver is performed the ischemic of the organ and perfusion with a preservation solution for the constitutional and functional maintenance and preserved at a cold storage of about 4°C to regulate metabolism. It has been known a UW (University of Wisconsin) solution and so on, as a cold preservation solution for liver (Documents 1 and 2).

Since the ischemia and perfusion with a preservation solution of an isolated liver are fundamentally harmful for the transplantation graft, there arises the problem of liver function disorder caused by the cold preservation ischemic reperfusion. In a cold preservation ischemic liver, it is explained that in general, the liver cells frequently suffer from damages mediated by a free radical or an inflammation-inducing substance and these damages cause liver dysfunction such as postgrafting thrombus formation and microcirculatory disorder. Since the problem of the depression of a liver to be transplanted due to cold preservation is a factor, which seriously affects the graft survival in the recipient, it is therefore strongly required to develop an effective method for overcoming this problem. Several methods have been proposed to approach the problem of the depression of in liver caused by the cold preservation ischemic reperfusion (see, for example, Document 3). However, the mechanism of the induction of the depression of liver during cold preservation still remains mostly unknown. As discussed above, the demand for isolated livers to be transplanted has been rapidly increasing in recent years and various preservation methods are under study. However, no drastic solution for this problem has been established hitherto.

It has never been reported that liver function ameliorating agents known as therapeutics for liver diseases (for example, STRONGER NEO-MINOPHAGEN C (registered trademark) as will be described hereinafter) would contribute to the improvement of the depression of liver occurring during cold preservation. Although there have been a number of reports on therapeutic agents for liver diseases, which may relate to the improvement in liver functions, these reports merely indicate clinical findings in the symptomatic utilization of these agents. Meanwhile, ALT (alanine aminotransferase), AST (aspartate aminotransferase) and so on, have been clinically employed as a liver test indication. This test method is that the concentrations of components, which are observed in the blood as the results of damages in liver cells, are measured. Thus, these indicators do not directly reflect liver functions *per se* in a precise sense.

In living donor liver transplantation, when a donor who may match for a recipient appears, the organ adaptabilities such as blood type, HLA type and lymphocyte cross-match are determined in a preliminary step. Even though these adaptabilities are confirmed, the transplantation cannot be successfully carried out unless the donor's liver per se is in good health. In particular, since the survival rate of a fatty liver after transplantation is low, the fatty liver is considered not to satisfy the adjustment requirement of the transplantation. Although liver generally contains somewhat lipids such as neutral fats and cholesterols, there are many cases that a fatty liver under the progress of obesity in which fat droplets in liver cells amount to 1/3 or more of the liver cells in the liver lobule is not evaluated to be used in transplantation. Since there is no drug or therapy, which has an immediate effect for treatment of fatty liver, when a donor is diagnosed as fatty liver, the donor is required to reduce the neutral fat accumulated in the liver through a diet or exercise program. In the case where a recipient does not have much time to live, a donor has to endure a sort-term strict diet, and is forced to bear a serious burden of providing the liver. However, unless the rate of the liver fat of the donor sufficiently reduces, transplantation cannot be performed. According to statistics, nearly 30% of adult candidates for donor have signs of fatty liver. Recently, the increase of fatty liver causes a shortage of the donor.

### DISCLOSURE OF THE INVENTION

To clarify the mechanism of non-necrotic graft dysfunction caused by the cold preservation ischemia, the present inventors have conducted intensive studies such as changes in the amounts of expulsion of bile and components of the bile relevant to the above mechanism. Further, they have studied the application of an organ function maintaining or ameliorating solution, which comprises a pharmacologically active substance for maintaining or ameliorating the liver functions under the cold preservation ischemia, and a method of preserving an organ. The present invention aims at, for example, properly maintaining the liver functions of a normal liver. According to the consideration of the inventors, non-necrotic dysfunction without any abnormal finding such as cell damage is a problem during the period required for organ preservation (usually from 2 to 24 hours) under the cold preservation ischemic conditions. In this regard, there was a particular problem, which was different from problems observed in the existing merely therapies for liver diseases. Furthermore, according to the present invention, the pharmacologically active substance unexpectedly inhibits depression in the ability of expelling a bile in a cold preservation ischemic liver and suppresses a change of ratio of components of the bile, as described in EXAMPLES hereinafter.

Moreover, as described above, a fatty liver is regarded as unsuitable for transplantation in these days and, therefore, a donor is required to be forced to bear a serious burden of reducing a rate of fatty liver. However, it is considered that the graft survival ratio depends not substantially on the fat percentage level of a fatty liver but rather on the depression of the liver per se in practice. If an appropriate pretransplantation treatment for a donor ensures the transplantation of a fatty liver, therefore, the burden on the donor can be seriously reduced. Thus, the present inventors have studied on an organ preservation method involving a pretransplantation treatment using a pharmacologically active substance, which is effective for reducing or resolving the burden of therapeutic programs and so on, of a donor diagnosed as a fatty liver, and, further, resolving the problem of the donor shortage.

Accordingly, the present invention provides an organ function maintaining or ameliorating solution used for maintaining or ameliorating the functions of an organ isolated for organ transplantation so that the functions of the transplanted organ can be more effectively restored after the transplantation, compared with existing organ preservation solutions, thereby promoting the satisfaction of various requirements in the art.

### BRIEF DESCRIPTION OF THE DRAWINGS

[Fig. 1] Fig. 1 is a graph showing bile flow to time for reperfusion.

[Fig. 2] Fig. 2 is a graph showing concentrations of the total bile salt in the expelled bile.

[Fig. 3] Fig. 3 is a graph showing the phospholipids concentrations in the expelled bile.

[Fig. 4] Fig. 4 is a graph showing the bile salt /phospholipids ratio in the expelled bile.

[Fig. 5] Fig. 5 is a graph showing the glutathione concentration in the expelled bile.

[Fig. 6] Fig. 6 is a graph showing bile flow to time for reperfusion.

[Fig. 7] Fig. 7 is a graph showing concentrations of the total bile salt in the bile expelled 20, 40 and 60 minutes after the initiation of the reperfusion.

[Fig. 8] Fig. 8 is a graph showing the phospholipids concentrations in the bile expelled 20, 40 and 60 minutes after the initiation of the reperfusion.

[Fig. 9] Fig. 9 is a graph showing the glutathione concentrations in the bile expelled 20, 40 and 60 minutes after the initiation of the reperfusion.

[Fig. 10] Fig. 10 is a chart showing the dosing schedules in EXAMPLE.

[Fig. 11] Fig. 11 is a graph showing changes of bile flow to time for reperfusion in a reperfusion experiment.

[Fig. 12] Fig. 12 is a graph showing the bile salt /phospholipids ratio in the bile.

[Fig. 13] Fig. 13 is a graph showing the glutathione concentration in the bile.

### DETAILED DESCRIPTION OF THE INVENTION

To clarify the mechanism of the depression of the liver under cold preservation ischemia, the present inventors conducted intensive studies and surprisingly found out that a specific therapeutic agent for liver diseases is effective for suppressing the depression of a cold preservation ischemic liver. Based on this novel use, they have developed an organ function maintaining or ameliorating solution and a method of preserving an organ by using thereof. The present inventors have further found out that when a donor has a fatty liver, the liver functions can be improved and the function of the transplanted liver can be effectively restored by administering a specific pharmacologically active substance before transplantation, and accomplished the organ preservation method of the present invention.

Accordingly, the present invention provides an organ function maintaining or ameliorating solution for maintaining or ameliorating the functions of an organ provided for organ transplantation, comprising a pharmacologically active substance comprising glycyrrhizic acid, glycyrrhetinic acid or a pharmacologically acceptable salt or derivative thereof in an organ preservation solution.

In an embodiment of the present invention, the pharmacologically active substance used in the present invention includes a substance extracted from licorice (*Glycyrrhiza glabra),* STRONGER NEO-MINOPHAGEN C (registered trademark) and GLYCYRON Inj. No.1 (registered trademark).

In another embodiment of the present invention, the organ function maintaining or ameliorating solution of the present invention may be used for maintaining or ameliorating the functions of an organ provided for transplantation, which is an organ isolated from a living body (including a brain-dead body) or a dead body, during the preservation and/or after the transplantation of the organ.

In another embodiment of the present invention, the organ preservation solution used in the organ function maintaining or ameliorating solution of the present invention includes UW (University of Wisconsin) solution, Euro-Collins solution (Document 4), Celsior solution (Document 5), HTK (Histidine-Tryptophan-Ketoglutarate) solution (Document 6) and Kyoto Solution (Document 7).

According to the present invention, a method of preserving an organ isolated for organ transplantation comprising (a) perfusing the organ function maintaining or ameliorating solution of the present invention to the isolated organ to cause ischemia and (b) immersing the organ in the organ function maintaining or ameliorating solution during the time until the organ is transplanted into a recipient is provided.

In an embodiment of the present invention, an organ preservation method further comprising a step of administering a pharmacologically active substance to a donor prior to the isolation of the organ to be used in organ transplantation from the living donor's body to maintain or improve the function of the organ is provided.

### MODE FOR CARRYING OUT THE INVENTION

The organ function maintaining or ameliorating solution of the present invention can be prepared by adding a required amount of a specified pharmacologically active substance to a physiologically acceptable organ preservation solution adjusted an equivalent osmotic pressure as body fluid. More specifically, the present invention relates to an organ function maintaining or ameliorating solution for maintaining or ameliorating the functions of an organ provided for organ transplantation, comprising a pharmacologically active substance comprising glycyrrhizic acid, glycyrrhetinic acid or a pharmacologically acceptable salt or derivative thereof in an organ preservation solution. The invention also relates to a method of preserving an organ isolated for organ transplantation comprising (a) perfusing the pharmacologically active substance to the isolated organ to cause ischemia and (b) immersing the organ in the organ function maintaining or ameliorating solution during the time until the organ is transplanted into a recipient.

### (1) Organ function maintaining or ameliorating solution

According to the present invention, an organ function maintaining or ameliorating solution for maintaining or ameliorating the functions of an organ provided for organ transplantation, comprising a pharmacologically active substance comprising glycyrrhizic acid, glycyrrhetinic acid or a pharmacologically acceptable salt or derivative thereof in an organ preservation solution is provided.

The term "organ function maintaining or ameliorating" as used herein refers to a maintain, improvement, restoration, suppression of depression and so on, of the functions of at least one organ among (a) an organ before the isolation from a donor, (b) an isolated organ before the transplantation, and (c) a transplanted organ into a recipient. In the case that the provided organ is a liver, the term mainly means ameliorating the liver functions by: suppressing the depression of the expulsion of the bile in a cold preservation ischemia liver isolated for transplantation; suppressing an undesirable change in components of the bile, particularly, suppressing the depression of the expulsion of important components of the bile such as glutathione conjugate (i.e., recovery of the detoxication ability); suppressing the depression of the expulsion of phospholipids; and suppressing an undesirable increase in the ratio of bile salts to phospholipids (the bile salt/phospholipids ratio).

The "pharmacologically active substance" used in the present invention includes glycyrrhizic acid, glycyrrhetinic acid or a pharmacologically acceptable salt or derivative thereof (hereinafter sometimes referred to as "glycyrrhizic acid or the like"). The glycyrrhizic acid or the like used as the pharmacologically active substance described above is not limited to their origin, process method and so on, so long as it has the characteristics described herein. That is to say; glycyrrhizic acid or the like is examined to be obtained by extracting from licorice or using a genetic engineering technique or a chemical synthesis technique, but not limited above. Glycyrrhizic acid or the like extracted from licorice is preferred. The liquorice plant is a legume plant native to Europe and Asia, and a herbaceous perennial or a subshrub having a subterranean stem of 1 to 2 m in length and stolons. Glycyrrhizic acid salts extracted from licorice root have been used as edible sweeteners. They also have analgesic, antipyretic, anti-inflammatory and detoxifying effects and so on. The Glycyrrhizic acid can be extracted from licorice in accordance with, for example, the method described in Document 8. When a glycyrrhizic acid salt or the like may be used as a pharmacologically active substance, it may be used in a crude state, preferably, it is required to have a purity of 60% or more.

The term "pharmacologically acceptable salt or derivative" as used herein means a compound which can be synthesized generally by forming a basic salt or by subjecting a functional group in the compound to well known manipulations by those skilled in the art or to induce the cleavage of a modification *in vivo* so as to give the parent compound. The pharmacologically acceptable salts or derivatives of glycyrrhizic acid or glycyrrhetinic acid includes, but not limited, an ammonium glycyrrhizinate, an alkali metal glycyrrhizinate, an organic base salt of glycyrrhetinic acid, an alkali metal glycyrrhetinate, 3β-O-glucuronide glycyrrhetinate and stearyl glycyrrhetinate. Preferably, it includes an ammonium glycyrrhizinate, potassium glycyrrhizinate and sodium glycyrrhizinate, more preferably, monoammonium glycyrrhizinate. The alkali metal glycyrrhetinate includes potassium glycyrrhetinate and sodium glycyrrhetinate.

In an embodiment of the present invention, the pharmacologically active substance used in the present invention includes STRONGER NEO-MINOPHAGEN C (registered trademark) or GLYCYRON Inj. No.1 (registered trademark). These pharmacologically active substances, which are available from MINOPHAGEN PHARMACEUTICAL Co., Ltd., have been clinically employed as prescription drugs for crude monoammonium glycyrrhizinate-containing injections.

Table 1 shows typical components in STRONGER NEO-MINOPHAGEN C (registered trademark) (hereinafter sometimes called "SNMC").

**Table 1 Composition of SNMC**

| Composition | | 20 mL |
|---|---|---|
| | Monoammonium glycyrrhizinate | 53 mg |
| Main Component | (as glycyrrhizic acid) | (40 mg) |
| | Glycine | 400 mg |
| | L-Cysteine hydrochloride | 2 mg |

In the case of using SNMC as the pharmacologically active substance, it is considered that the glycyrrhizinate, which is one of the active components thereof, contributes to the effects and advantages of the present invention. From this viewpoint, the pharmacologically active substance, which can be used in the organ function maintaining or ameliorating solution of the present invention, includes other glycyrrhizic acid-containing compositions having the same main components as the said SNMC.

On the other hand, GLYCYRON Inj. No.1 (registered trademark) is an allergy medicine and mainly effective for drug exanthem and the like. Regarding the composition of GLYCYRON Inj. No.1 (registered trademark), 53 mg of crude monoammonium glycyrrhizinate (40 mg as glycyrrhizic acid) as a main component is contained per 2 ml of this agent.

In an embodiment of the present invention, the organ function maintaining or ameliorating solution of the present invention can be used for maintaining or ameliorating the functions of an organ provided for transplantation, which is an organ isolated from a living body (including a brain-dead body) or a dead body, during the preservation and/or after the transplantation of the organ. The organ to which the organ function maintaining or ameliorating solution of the present invention is applicable is not particularly limited, so long as it is an organ provided for transplantation. Preferably, the organ is liver, kidney, pancreas, lung or heart, more preferably, liver, kidney, pancreas or lung and most preferably, liver.

Further, the organ for transplantation is not particularly limited, preferably originates from a mammalian source, more preferably a human, swine, bovine or goad source and most preferably a human source.

The organ preservation solution used in the organ function maintaining or ameliorating solution of the present invention is not particularly limited, so long as it can be used as an existing organ preservation solution. The organ preservation solution includes, preferably, UW solution, Euro-Collins solution, Celsior solution, HTK solution and Kyoto Solution, more preferably, UW solutions and Celsior solution, and most preferably, UW solution.

The organ function maintaining or ameliorating solution of the present invention can be prepared by adding a desired amount of the pharmacologically active substance to the organ preservation solution. The concentration of the pharmacologically active substance in terms of SNMC (an injection) in the organ preservation solution is preferably from 1 to 30%, more preferably from 2 to 20% and most preferably from 2 to 10%. For example, in the case of using an organ function maintaining or ameliorating solution prepared by adding SNMC to a UW solution employed as the organ preservation solution, as specifically discussed in EXAMPLES hereinafter, the UW solution: SNMC ratio by volume preferably ranges from 98:2 to 80:20, more preferably about 95:about 5. In addition to the pharmacologically active substance, it is possible to add an optional component such as an antibiotic, a steroid, a hormone agent, a pH regulator and so on, so long as such a component is compatible with the organ function maintaining or ameliorating solution of the present invention.

The organ function maintaining or ameliorating solution of the present invention is not limited to use for preservation of an isolated organ, and can be used in steps, which are essentially required to maintain or improve the organ functions in a series of the course of the organ transplantation. More specifically, the organ function maintaining or ameliorating solution of the present invention can be used as a preservation solution, a washing solution, an immersion solution, a perfusate, a rinsing solution and so on, for the organ.

### (2) Organ preservation method

As described above, the organ, which can be treated with the organ function maintaining or ameliorating solution of the present invention may be provided by either a living donor (including a brain-dead donor) or a dead donor. The organ function maintaining or ameliorating solution of the present invention is effectively applicable to the case where an organ should be preserved over such a long time as bringing about a fear of the occurrence of non-necrotic graft dysfunction in the cold preservation ischemia state.

The present invention provides an organ preservation method using the above-described organ function maintaining or ameliorating solution. More specifically, the present invention relates to a method of preserving an organ isolated for organ transplantation which comprises: (a) perfusing the said organ function maintaining or ameliorating solution to the isolated organ to cause ischemia; and (b) immersing the organ in the organ function maintaining or ameliorating solution during the time until the organ is transplanted into a recipient.

The organ preservation method of the present invention is not particularly limited so long as the organ function maintaining or ameliorating solution as defined herein is employed, and typically comprises perfusing the above organ function maintaining or ameliorating solution to the organ isolated from a donor to cause ischemia and immersing the organ in the organ function maintaining or ameliorating solution during the desired time until the organ is transplanted into a recipient.

In the organ preservation method of the present invention, the time of immersing the isolated organ in the organ function maintaining or ameliorating solution (the preservation time) is not limited, but generally lasts from the isolation of the organ from the donor to the transplantation into the recipient. The preservation time differs depending on the kind of organ, for example, in the case of liver, the preservation time is preferably 24 hours or shorter, more preferably from 2 to 24 hours and more preferably form 2 to 16 hours. A person skilled in the art can appropriately control the preservation time of an organ by considering the kind of the organ, the transplantation timing and so on. The organ function maintaining or ameliorating solution used in the organ preservation method of the present invention is preferably cooled before using. Preservation of an organ using the organ preservation method of the present invention is specifically described in EXAMPLE 1 and so on, hereinafter.

As described above, a donor having a fatty liver is regarded as unsuitable for liver transplantation. Thus, the present inventors have conducted intensive studies on the mechanism of depression of the non-necrotic liver functions during the liver transplantation and perfusion, and have attempted to improve the transplantation compatibility of a fatty liver by noting such a depression. A liver isolated for living donor liver transplantation is treated by cold preservation ischemic perfusion and then preserved under cold storage. To evaluate, it is important for evaluation of the transplantation compatibility of a fatty liver to observe the depression of the liver caused by the cold preservation ischemic perfusion.

From this viewpoint, in an embodiment of the present invention, the organ preservation method as described above may further comprise an additional step of administering a pharmacologically active substance to a donor prior to the isolation of the organ used in organ transplantation from the living donor's body to thereby maintain or improve the functions of the organ. The step of administering a pharmacologically active substance is not particularly limited, and can apply an administration route appropriate for the pharmacologically active substance employed. The administration of the pharmacologically active substance is, preferably, intravenous, oral, transdermal, intramuscular, nasal, inhalation or rectal administration, more preferably, intravenous or oral administration, most preferably, intravenous administration. Those skilled in the art can control the selection of a pharmacologically active substance and the dose amount and dosing period thereof can be based on the severity of the disease. The dose of the pharmacologically active substance is, for example, in the case of using SNMC (an injection), ranges from about 0.1 to 2 mL/day per kg of the donor's body weight, preferably from about 0.3 to 1.7 mL/day and more preferably 1 mL/day. The number of dosing is not particularly limited before the isolation of the organ, and the pharmacologically active substance can be administered either everyday or every other day. Further, the period of the administrating pharmacologically active substance to a donor may be everyday for at least 2 weeks, preferably. The single dose to an adult donor ranges from 20 to 100 mL per day, preferably from 40 to 100 mL and more preferably about 60 mL. The dose and dosing time are appropriately controlled depending on the extent of the progress of the fatty liver in the donor and the acceptable administration time (for example, considering the life expectancy of the recipient).

The organ disease applied the organ preservation method of the present invention is not particularly limited so long as the maintenance or improvement of the organ functions can be expected by administering a pharmacologically active substance in the disease. The disease includes, preferably, a liver disease, a kidney disease, a pancreas disease and a lung disease, more preferably, a liver disease and a kidney disease, and most preferably, a liver disease. The liver disease includes, preferably, fatty liver and hepatitis and, more preferably, fatty liver.

When an isolated liver is preserved by using the organ preservation method of the present invention, the maintenance or improvement of the liver functions can be evaluated by measuring the flow rate of the bile from the liver and the bile salt concentration, the phospholipids concentration, the bile salt/phospholipids ratio, the glutathione concentration and so on, in the expelled bile.

As described above, in addition to the pharmacologically active substance, the organ function maintaining or ameliorating solution of the present invention may contain an optional component such as an antibiotic, a steroid, a hormone agent, a pH regulator and so on, so long as such a component is compatible with the above solution of the present invention.

As shown in EXAMPLE 1 hereinafter, from the results measured functions of liver in an organ preservation solution added SNMC (containing glycyrrhizic acid) and the liver in the solution added glycyrrhizic acid, since it was shown that both additives have similar effects of restoring the liver functions, it was indicated that glycyrrhizic acid is the major pharmacologically active substance.

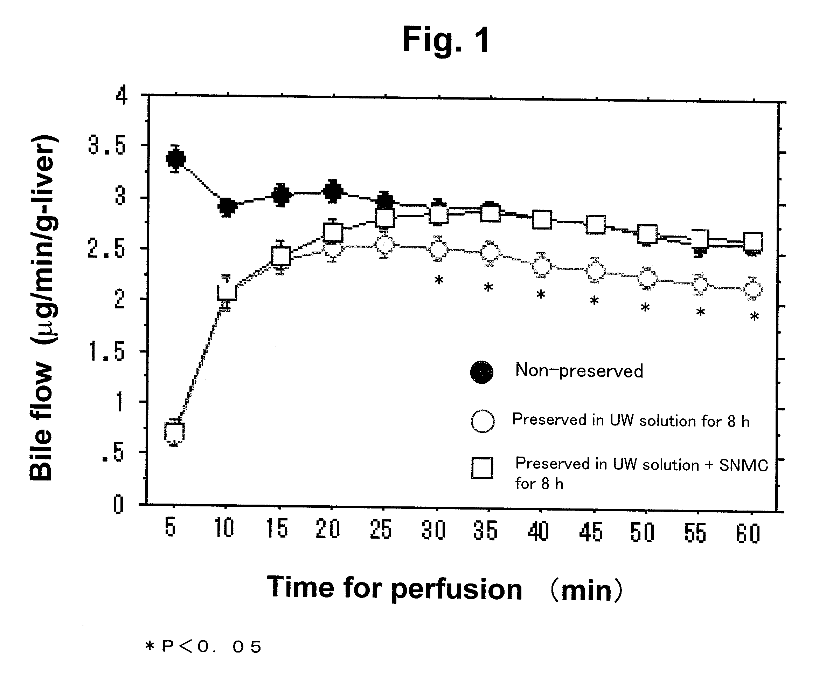

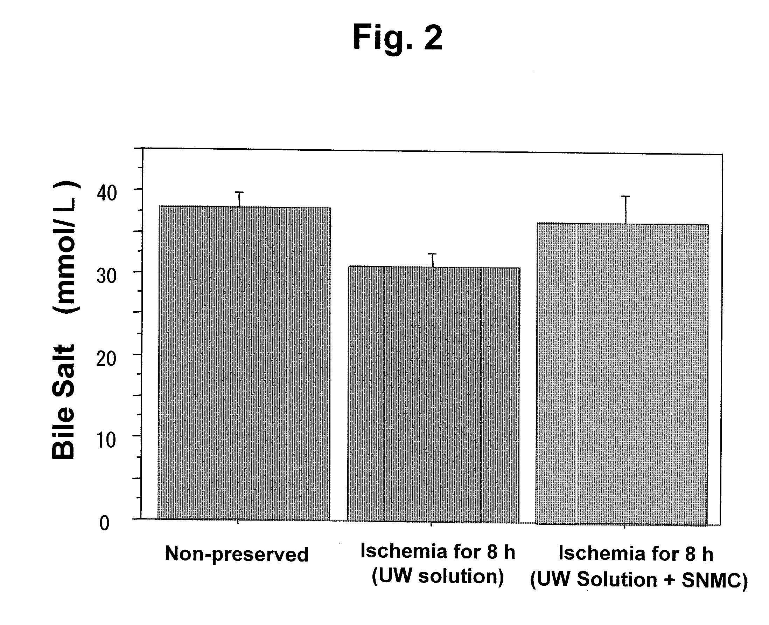

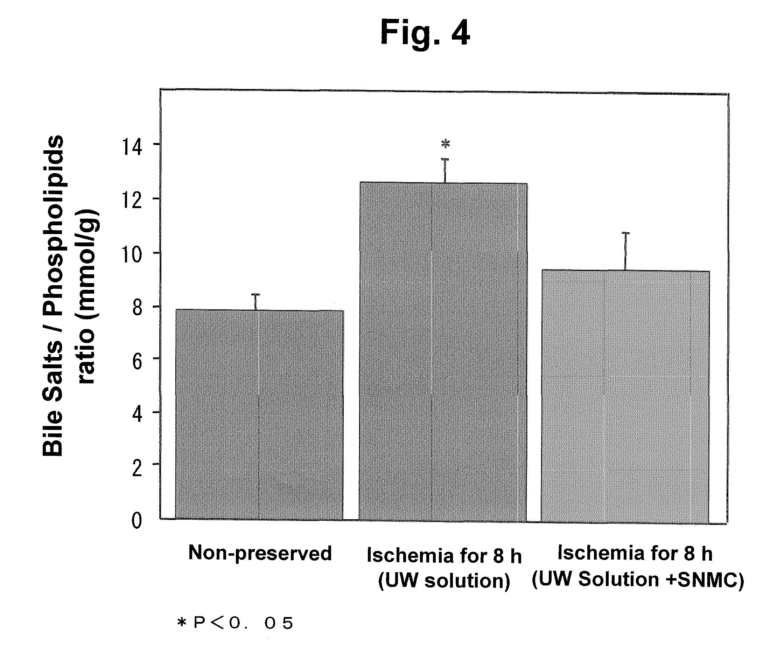

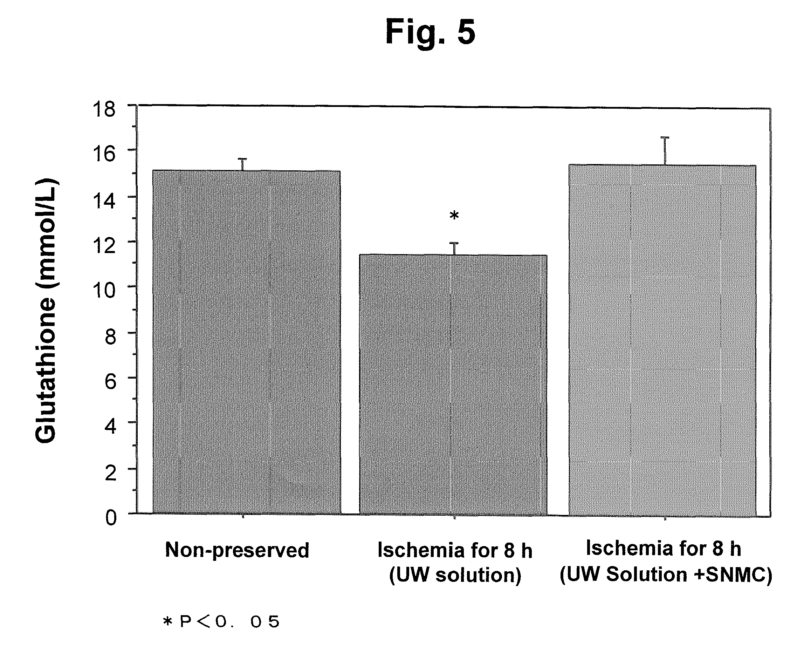

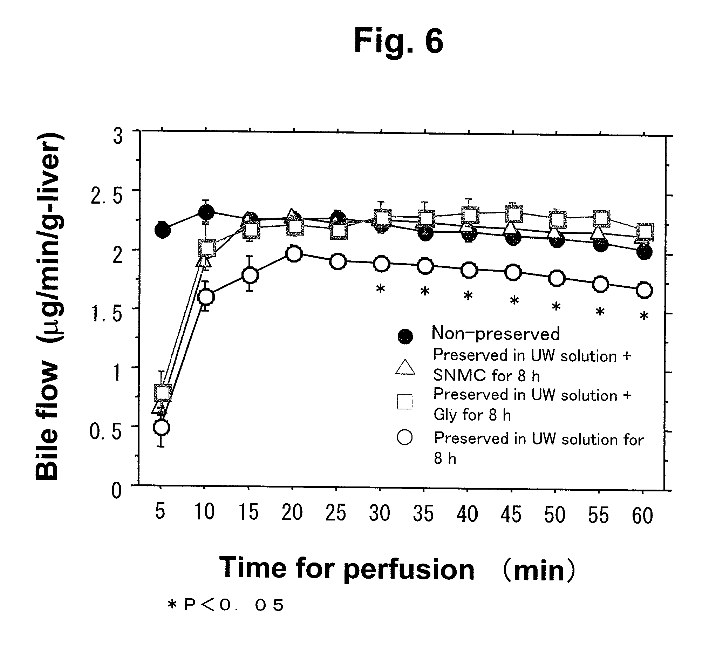

As shown in EXAMPLE 1 hereinafter, the effects of the present invention are that the bile flows were restored to a same level compared with a non-preserved liver when the bile flow was measured in livers preserved by the organ function maintaining or ameliorating solution of the present invention of which UW solution contains monoammonium glycyrrhizinate as SNMC or GLYCYRON Inj. No.1. Compared with a liver preserved in the UW solution alone, the bile flows were remarkably restored (Figs. 1 and 6). Further, bile salts and phospholipids, which are indicators of the liver function restoration, were remarkably restored by the present invention (Figs. 2, 3, 7 and 8). The bile salt /phospholipids ratio could be restored to a same level compared with non-preserved control (Fig. 4). It was also indicated that the expulsion amount of the glutathione conjugate was remarkably restored (Figs. 5 and 6).

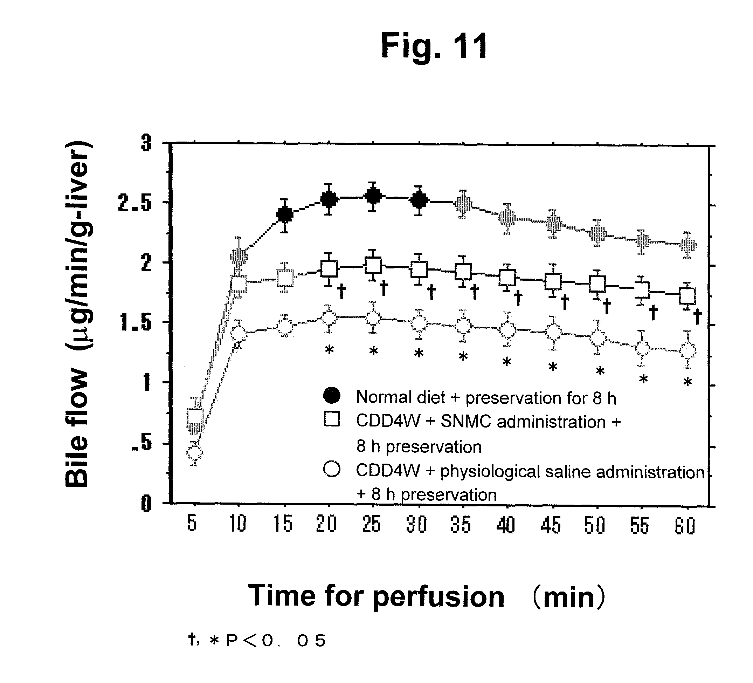

As shown in EXAMPLE 2 hereinafter, the administration of a specific pharmacologically active substance to fatty liver model animals before an isolation surgery contributed to significant improvements in the functions, for example, restoration of the bile expulsive ability and the concentrations of major bile components, in the fatty livers after the isolation, preservation under cooling and reperfusion. It was confirmed that the administration of the specific pharmacologically active substance before the surgery contributed to improvements in the functions of the fatty livers isolated after the administration (Figs. 11 to 13). These results indicate that the present invention can enhance the transplantation compatibility of a fatty liver.

### EXAMPLES

The present inventions are described in greater detail on the basis of the EXAMPLES. However, the present invention should not be limited thereto. All animal experiments were carried out in accordance with the guideline of Tokyo Medical and Dental University. Unless otherwise noted, livers isolated from male Wistar rats (250 to 300 g) and subjected to separate perfusion were employed in these experiments.

### Experiment 1: Restoration of liver functions after liver transplantation

(1) Isolated livers were perfused at the first pass of 2.75 mL/min/g liver using Krebs-Henseleit Buffer. The amount of sodium taurocholate (SIGMA) in the perfusate was at the physiological level (30 mM).

Cold preservation was conducted for 8 hours at 4°C by using 100mL of UW solution (VIASPAN; FUJISAWA PHARMACEUTICALS Co., Ltd. (now ASTELLAS PHARMA Inc.)). A preservation solution was prepared by adding 5 mL of SNMC (MINOPHAGEN PHARMACEUTICAL Co., Ltd.) to 95 mL of the UW solution. The preservation solution of the above composition was also employed in rinsing (preconditioning) before the reperfusion.

In this experiment, the livers were reperfused at 37°C after the cold preservation. Then, the bile flow and the components of the expelled bile were analyzed. Specifically, the bile flow was determined from the amount of carboxyfluoresceine dye expelled from liver cells, into which the dye was incorporated, to the bile duct. Further, phospholipids and glutathione contained in the bile were collected and then, quantified. Control samples of the non-preserved group (preservation time=0) and comparative samples of the group with 8 hours preservation in 100 mL of the UW solution were also subjected to the reperfusion experiment under the same conditions.

Fig. 1 shows changes in the bile flow during reperfusion. Figs. 2 to 5 show the flow rate of expelled bile, the concentrations of bile salts, phospholipids and glutathione in the bile and the bile salt/phospholipids ratio, respectively.

As shown in Fig. 1, the ischemic livers preserved under cooling for 8 hours (open circles) showed the restoration of the liver function level by the reperfusion for about 20 minutes from the cold preservation state. Further, the bile flow rapidly increased, however, did not attain the control level (close circles). In contrast thereto, the ischemic livers preserved under cooling for 8 hours in the solution containing SNMC (open squares) showed the complete restoration of the bile flow to the control level by the reperfusion.

With respect to the components of the expelled bile, the total amount of the expelled bile salts and the glutathione concentration in the bile expelled from the livers preserved under cooling for 8 hours in the solution containing SNMC were each comparable to the control level (Figs. 2 and 5), respectively. These differences in the amount of the expelled bile salts and the glutathione concentration indicate the suppressive effect of SNMC against depression of the liver occurring during the cold preservation ischemia for 8 hours by the existing method. Thus, the preservation solution containing SNMC has a remarkable prophylactic effect of decrease in the ability for expelling bile expulsion, in particular, for expelling the glutathione conjugate.

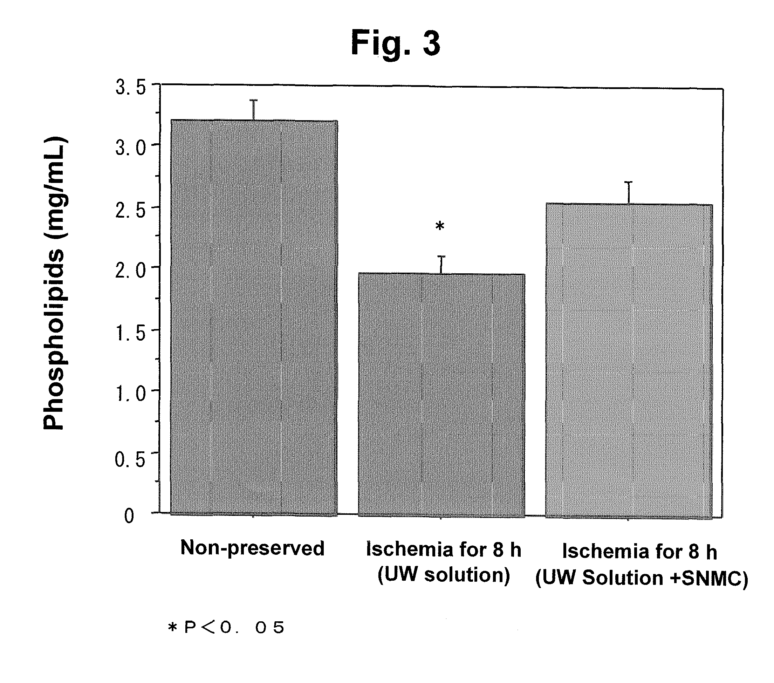

With respect to the livers preserved under cooling for 8 hours in the UW solution, change of bile salt concentration was little compared with the control (Fig. 2), but phospholipids concentration largely decreased (Fig. 3). The bile salt/phospholipids ratio was extremely elevated in the cold preservation ischemia for 8 hours in the UW solution. In the cold preservation ischemia for 8 hours in the solution containing SNMC, on the other hand, it is shown a remarkably suppressive effect against an increase in the bile salt /phospholipids ratio (Fig. 4).

Based on these results, it was confirmed that the preservation solution containing SNMC is effective in suppressing the depression of cold preservation ischemic liver.

(2) In the case of preparing the organ function maintaining or ameliorating solution of the present invention using GLYCYRON Inj. No.1 (hereinafter sometimes called "Gly"), change in bile flow, the total concentration of the expelled bile salt and the concentrations of phospholipids and glutathione in the organ after a desired preservation period were measured. Gly was employed in an amount of 1/10 times as much as SNMC (the same amount in terms of glycyrrhizic acid).

The individual tests providing the indicators of the restoration of the liver functions as described above were conducted by the same methods as in the above (1) but the perfusion and reperfusion of the isolated liver were conducted at a flow rate of 2.75 mL/min/g liver.
Fig. 6 shows change in the bile flow during the reperfusion, and in the case of adding Gly to the UW solution (open squares), the bile flow was restored to the same level as in the case of adding SNMC. The bile flow completely restored to the control level (close circles) by using Gly.

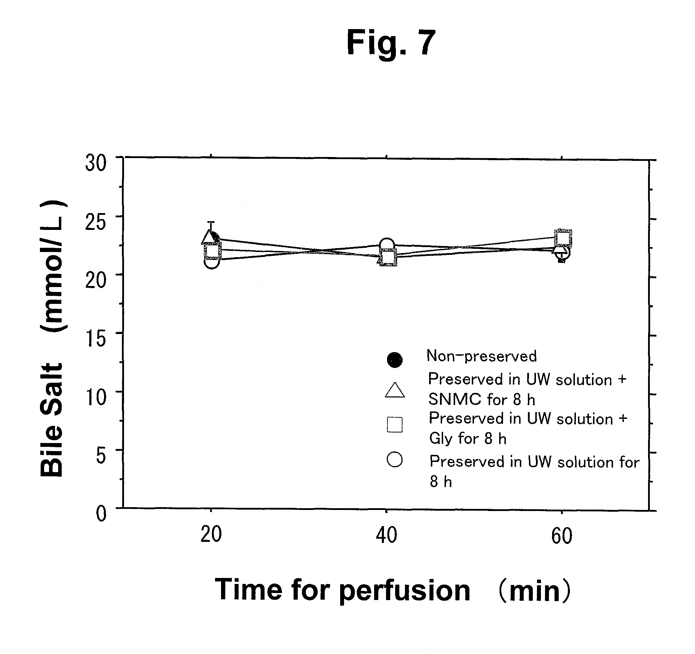

Fig. 7 shows the total amounts of bile salts in the bile expelled 20, 40 and 60 minutes after the initiation of the reperfusion. In the case of adding Gly, the total expelled amount of bile salts could be induced to the same level as that of the control.

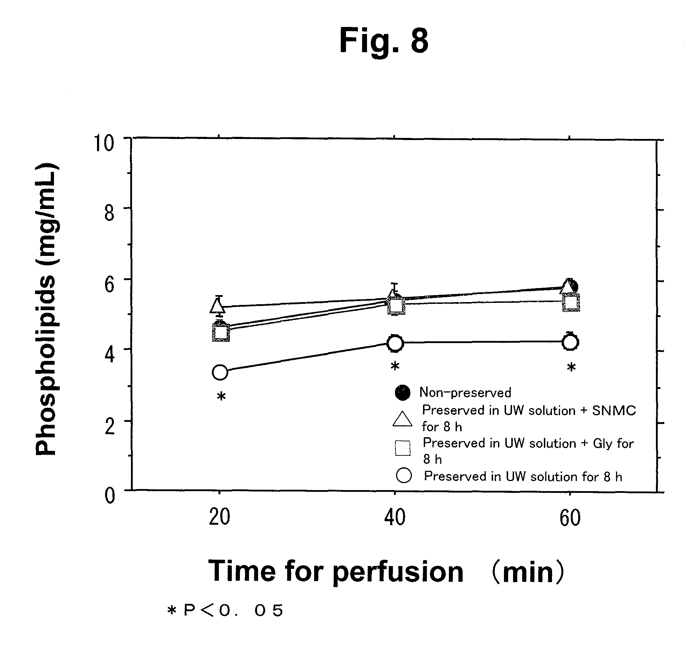

Fig. 8 shows the phospholipids concentrations in the bile expelled 20, 40 and 60 minutes after the initiation of the reperfusion. The phospholipids concentration could not be sufficiently restored by using the UW solution alone. In the case of adding Gly, however, the phospholipids concentration could be restored to the same level as control.

As well as the above (1), the bile salt/phospholipids ratios were calculated. Although the bile salt/phospholipids ratio was largely increased by the cold preservation ischemia in the UW solution for 8 hours, the increase in the bile salt /phospholipids ratio was remarkably suppressed in the case of adding Gly (data not shown).

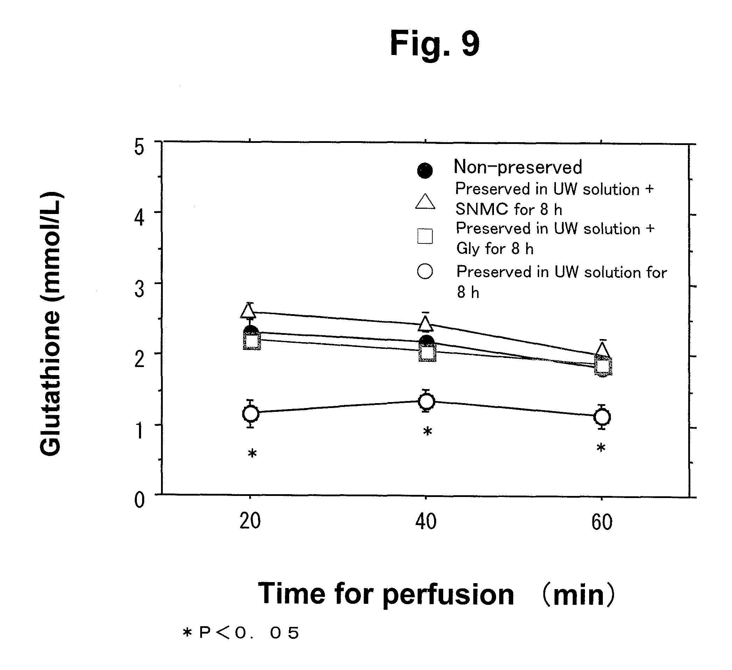

Fig. 9 shows the glutathione (GSH) concentrations in the bile expelled 20, 40 and 60 minutes after the initiation of the reperfusion. In the case of adding Gly, the GSH concentration could be restored to the same level as the control.

Based on these results, it was confirmed that the organ function maintaining or ameliorating solution containing Gly is effective in suppressing the depression of cold preservation ischemic liver.

### EXAMPLE 2: Pretransplantation treatment of fatty liver and restoration of liver functions after transplantation

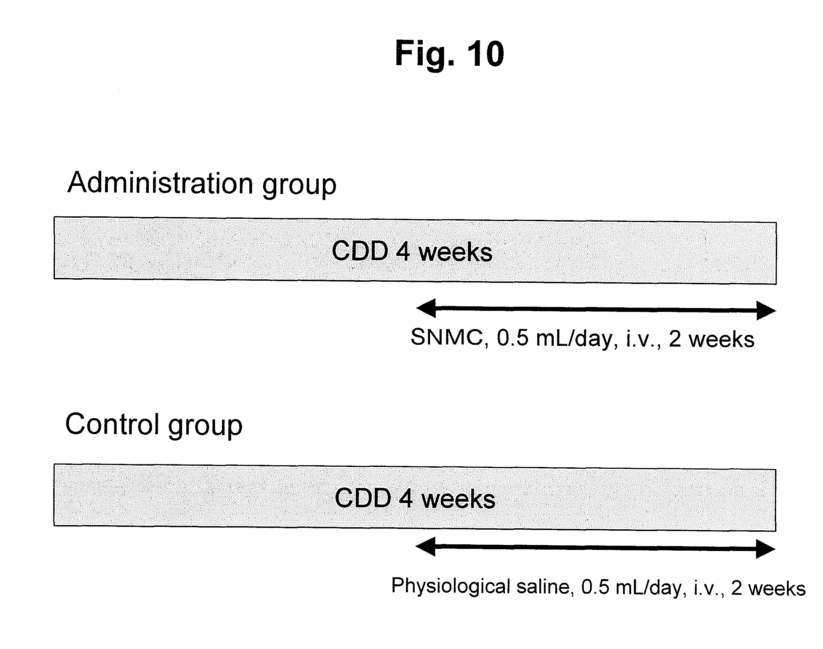

Rats suffer from fatty livers by feeding a choline-deficient diet (ORIENTAL YEAST Co., Ltd.). As shown in Fig. 10, the rats were divided into 2 groups. Then, all rats were fed on the choline-deficient diet for 4 weeks. During the latter two weeks, 0.5 mL/day of SNMC was intravenously injected everyday to each of the rats in one group, while physiological saline was given to the rats of the other group in the same dose by the same method.

Livers isolated from the rats were subjected to the perfusion experiment. The perfusion of the livers was conducted at the first pass of 2.75 mL/min/g liver using Krebs-Henseleit Buffer. The amount of sodium taurocholate (SIGMA) in the perfusate was at the physiological level (30 mM). The cold preservation was conducted for 8 hours at 4°C with the use of 100 mL of the UW solution.

To measure the liver functions, the livers preserved under cooling for 8 hours were reperfused at 37°C, and then, the bile flow and the components of the expelled bile were measured. The bile flow was determined from the amount of carboxyfluoresceine dye expelled from liver cells, into which the dye had been incorporated, into the bile duct, and from the bile collected, the bile salt and phospholipids concentrations therein were determined.

Fig. 11 shows the flow rate in the reperfusion after preserving for 8 hours. The rats fed on the normal diet did not suffer from fatty liver. Accordingly, these normal livers expelled bile at a high level during the reperfusion. In contrast thereto, the livers of the rats fed on the choline-deficient diet had been converted into fatty livers and thus, the bile flow showed to be lower than the normal livers. Namely, fatty livers show depression of the fundamental liver functions. The group administrated SNMC among the fatty liver group showed a higher bile flow than the group administrated physiological saline. Thus, it is shown that SNMC has an effect of ameliorating bile flow.

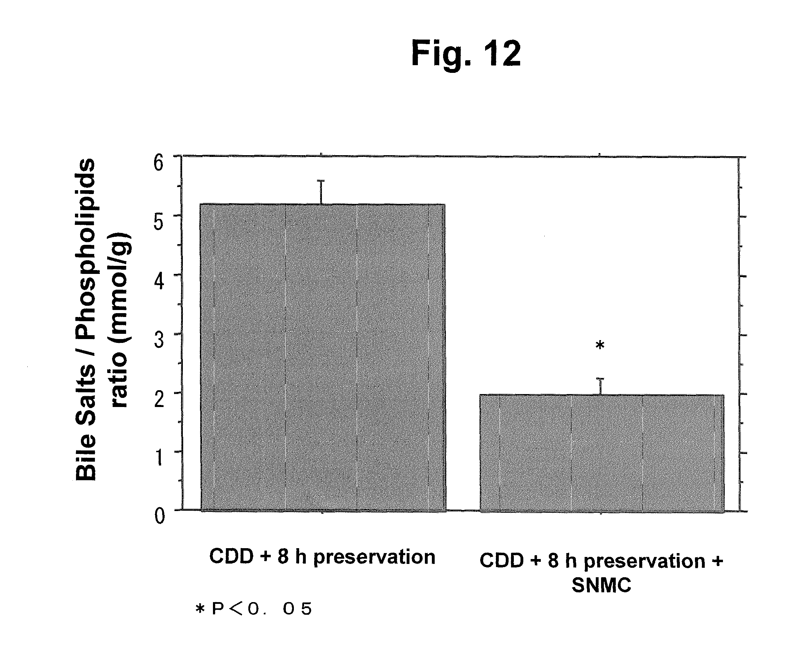

Fig. 12 shows the bile salt /phospholipids ratio. With respect to the bile salt /phospholipids ratio, the bile salt /phospholipids ratio of the non-administration group was extremely high while such an increase was suppressed in the administration group. These results indicate that the administration of SNMC improves the bile salt /phospholipids ratio.

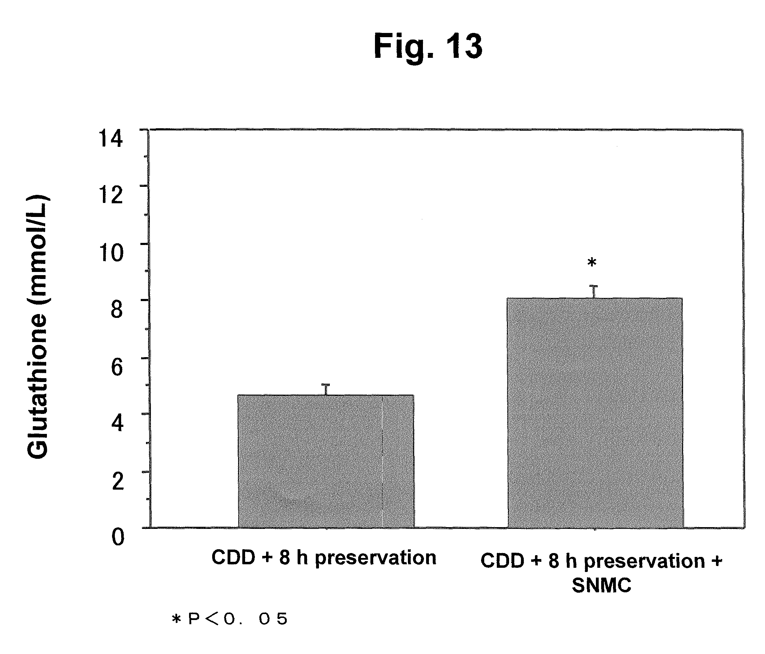

Fig. 13 shows the glutathione concentrations. From the results of the glutathione concentrations, it was indicated that the expulsion amount of the glutathione conjugate was remarkably restored.

Based on the results described above, it has been confirmed that the administration of SNMC to fatty liver models before the isolation surgery contributes to the improvement of the major functions of the liver isolated thereafter.

### REFERENTIAL DOCUMENTS

1. United States Patent 4,879,283
2. United States Patent 4,798,824
3. JP-A-2001-335401
4. Squifflet, J.P., Pirson, Y., Gianello, P., Van Cangh, P., and Alexandre G.P., Safe preservation of human renal cadavar transplants by Euro-Collins solution up to 50 hours, Transplant. Proc., vol.13, p.693-696 (1981)
5. Mohara, J., Morishita, Y., Takahashi, T., Oshima, K., Yamasishi, T., Takeyoshi, I., and Matsumoto, K., A comparative study of Celsior and University of Wisconsin solutions based on 12-hr preservation followed by transplantation in canine models, J. Heart Lung Transplant., vol. 18, p.1202-1210 (1999)
6. Erhard, J., Lange, R., Niebel, W., et al., Results of experimental liver transplantation in the minipig with HTK preserved organs. In: Engemann R, Hamelmann H, eds. Experimental and clinical liver transplantation. Amsterdam: Elsevier, 93, (1999)
7. Japanese Patent No.3253131
8. Kitagawa, I., Hori, K., Sakagami, M., Hashiuchi, F., Yoshikawa, M., and Ren, J., Saponin and sapogenol. XLIX. On the constituents of the roots of Glycyrrhiza inflata Batalin from Xinjang, China. Characterization of two sweet oleanane-type triterpene oigoglycosides, apioglycyrrhizin and araboglycyrrhizin. Chem., Pharm. Bull (Tokyo), vol. 41, p.1350-1357 (1993)

## Claims

1. An organ function maintaining or ameliorating solution for maintaining or ameliorating the functions of an organ provided for organ transplantation, comprising a pharmacologically active substance comprising glycyrrhizic acid, glycyrrhetinic acid or a pharmacologically acceptable salt or derivative thereof in an organ preservation solution.

2. The organ function maintaining or ameliorating solution according to claim 1, wherein the pharmacologically active substance comprising glycyrrhizic acid, glycyrrhetinic acid or a pharmacologically acceptable salt or derivative thereof is extracted from licorice.

3. The organ function maintaining or ameliorating solution according to claim 1 or 2, wherein glycyrrhizic acid, glycyrrhetinic acid or a pharmacologically acceptable salt or derivative thereof is selected from the group consisting of an ammonium glycyrrhizinate, an alkali metal glycyrrhizinate, an organic base salt of glycyrrhetinic acid, an ammonium glycyrrhetinate, an alkali metal glycyrrhetinate, 3β-O-glucuronide glycyrrhetinate and stearyl glycyrrhetinate.

4. The organ function maintaining or ameliorating solution according to claim 1, wherein the pharmacologically active substance is STRONGER NEO-MINOPHAGEN C (registered trademark) or GLYCYRON Inj. No.1 (registered trademark).

5. The organ function maintaining or ameliorating solution according to any one of claims 1 to 4, wherein the organ provided for organ transplantation is an organ isolated from a living body or a dead body and the solution aims at maintaining or ameliorating the functions of the organ during the preservation and/or after the transplantation.

6. The organ function maintaining or ameliorating solution according to any one of claims 1 to 5, wherein the organ provided for organ transplantation is liver, heart, lung, kidney or pancreas.

7. The organ function maintaining or ameliorating solution according to claim 6, wherein the organ provided for organ transplantation is liver.

8. The organ function maintaining or ameliorating solution according to any one of claims 1 to 7, wherein the organ preservation solution is UW solution, Euro-Collins solution, Celsior solution, HTK solution or Kyoto Solution.

9. A method of preserving an organ isolated for organ transplantation comprising the following steps:
(a) perfusing the organ function maintaining or ameliorating solution according to any one of claims 1 to 8 to the organ isolated from the donor to cause ischemia; and
(b) immersing the organ in the organ function maintaining or ameliorating solution during the time until the organ is transplanted into a recipient.

10. The organ preservation method according to claim 9 further comprising a step of administering a pharmacologically active substance to a donor prior to the isolation of the organ to be used in organ transplantation from the living donor's body to maintain or improve the function of the organ.

11. The organ preservation method according to claim 10, wherein the donor suffers from liver disease.

12. The organ preservation method according to claim 11, wherein the liver disease is fatty liver.
